# EUROPEAN PATENT SPECIFICATION

(11) **EP 1 450 154 B1**
(45) Date of publication and mention of the grant of the patent: **21.10.2009**
(21) Application number: 04101218.8
(22) Date of filing: 20.02.1997
(51) Int. Cl.: G01N 23/10, G01N 33/02

(54) **Non destructive x-ray inspection apparatus for food industry**
Zerstörungsfreie Röntgenstrahlungsuntersuchung für die Nahrungsmittelindustrie
Inspection par rayons X non destructive pour l'industrie alimentaire

(30) Priority: 15.03.1996 IT TO960203
(43) Date of publication of application: 25.08.2004
(62) Divisional of application: 97102770.1
(73) Proprietor: Dylog Italia S.p.A., 10126 Turin (IT)
(72) Inventor: Ocleppo, Rinaldo, 12043 Canale (CN) (IT)
(74) Representative: Robba, Pierpaolo

(56) References cited:
- DE-A1- 4 137 510
- US-A- 3 958 078
- US-A- 5 442 672

## Description

The present invention deals with a non-descructive X-ray inspection apparatus for food industry, particularly for glass vessels and/or metal cans.

It is known that, in the vast majority of cases, non-destructive X-ray inspection for glass vessels in food industry is carried out by single devices, located on one side of the row of said vessels to be inspected; said devices being substantially equipped with an emitter and a sensor between which the row of said vessels to be inspected moves.

Similarly known is the fact that with such single devices a 90-95% inspection is obtained of the product contained therein, said deficiency being due to the shadow area projected by the concavity that is present, more or less importantly, in the vessel bottom.

To be more precise, refer to a glass vessel containing jam or a similar product; the impairing concavity is the one on the bottom of these vessels; this projection overlaps the contaminating agents hiding them and making their recognition impossible.

In many cases this result has been deemed, wrongly, enough, because an inspection valid only at 95% created a lot of problems which it was attempted to solve through a few solutions, that proved however rather complicated and costly and not satisfactory enough.

Among the above solutions, mention can be made of a 45°-slant on the vertical emitter pipe plan, solution that only generated another shadow area in another point with respect to the traditional inspection apparatus placed on one side; moreover, though mitigating the darkening, it did not solve the problem, often due to high vessel tolerances. Furthermore, it can not be applied to cans.

Another solution being studied in the United Kingdom, provides two inspection apparata mutually placed at 90° on the vertical plan.

This latter solution seems to benefit from a partial reduction of the shadow areas, but it uses two inspection apparata and the system overall dimensions are rather big; the cost for the two apparata, their maintenance and the like are important cost elements that cannot be overlooked.

DE 41 37 510 illustrates a scanning system for investigating test object passing on a transport path for unauthorised contents. The system contains a source of radiation emitting two fan-shaped beams toward two corresponding linear sensors. The angle between the plane of at least one beam and the transport direction differs from 90 deg. A signal processing device process the signals of the sensors in order to provide a tridimensional view of the scanned object.

US 3 958 078 refers to an X-ray inspection apparatus for scanning articles containing food products. One of the embodiment illustrated provides for the presence of two independent X-ray sources optically coupled to corresponding sensors, the two scanning directions being mutually arranged at 90 deg. The two X-ray sources and the two sensors are four separate units.

US 5 442 672 illustrates an apparatus for reconstructing a tridimensional image of an object. This apparatus provides as well the presence of two independent X-ray sources coupled to corresponding sensors, mutually arranged at 90 deg.

EP 0 375 157 relates to an on-line X-ray inspection system comprising a source of X-rays providing a very thin beam of rays of general fan shape and, opposite to the X-ray source, detector means in the form of a linear array of photo-sensitive diodes.

Object of the present invention is that of providing a non-destructive X-ray inspection apparatus for food industry which solves the above-mentioned problem of how to remove the shadow area during the inspection of packaged vessels, by realising an improved, simple and practical apparatus, which allows to easily attain the predetermined object of a complete inspection of the sealed vessels.

The inspection apparatus of the present invention is composed of a static structure comprising two modular units mutually placed at 90° and placed at 45° with respect to a feeding line for vessels to be inspected; said inspection structure with two modular units each incorporating a corresponding emitter and sensor realizes a structure able to inspect 100% of a product contained in said vessels.

In addition, thanks to the fact that the structure comprises two modular units wherein each is equipped with an emitter and a sensor, the apparatus according to the present invention results more reliable and stable with respect to the apparata having four separate units, two emitters and two sensors, which are more complicated to get ready.

The advantages of the apparatus of the invention are as follows:
- modularity: therefore, with the same performance a starting price much less than the standard price, thus widening product markets;
- with two sensors a simultaneous failure of both of them is statistically impossible, whereby the user can go on working even in case one of the two sensors fails (inspecting the 95% of the product);
- there are practically no installation problems and related costs;
- there are no costs for mechanical spare parts, being the structure substantially static with obvious maintenance advantage;
- there are no problems of space, thus complying with the food industry needs whose lines are almost always full and without spaces for apparata not provided for when designing.

Further features and advantages will appear from the following detailed description of an embodiment of the invention, with reference to the accompanying drawings, provided as a non limiting example, in which:
- Fig. 1 is a schematic plan view of a prior art inspection apparatus, placed at 90° with respect to the line of vessels to be inspected;
- Fig. 2 is a schematic plan view of a prior art inspection apparatus, placed at 45° with respect to the line of vessels;
- Fig. 3 is a schematic sectional view of the apparatus of Fig. 1;
- Fig. 4 is a schematic plan view of an inspection apparatus according to the invention;
- Fig. 5 is a section of a glass vessel with the typical bottom concavity.

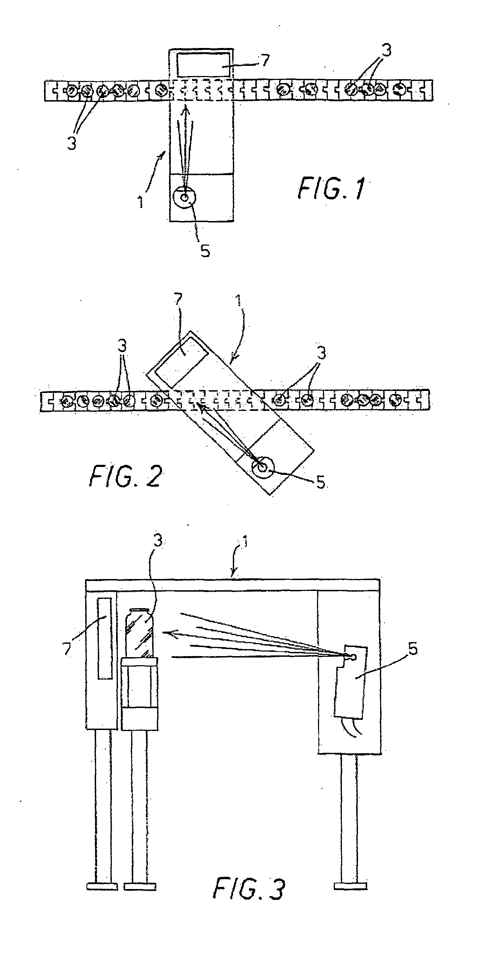

As appears from Figures 1 to 3, prior art inspection apparata are composed of a structure 1 bridging a row on glass vessels 3 to be examined that moves thereunder.

This structure includes an X-ray emitter 5 and a sensor 7, that collects rays emitted by the emitter 5 and displays what has been inspected.

If a glass vessel is taken into account, it can be clearly seen how the concavity 9 provided on the bottom of said vessel 3 impairs a complete inspection of the product in the vessel; should this concavity or internal projection 9 be missing, obviously there would be no problems.

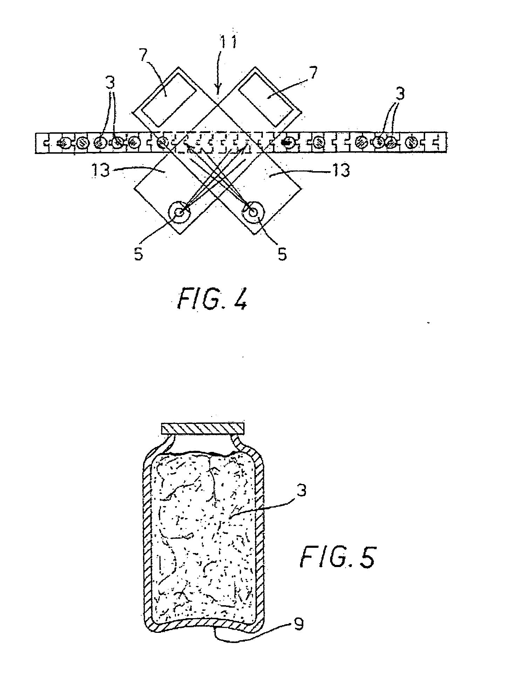

With the inspection apparatus of the invention shown in Fig. 4, instead, a static structure 11 is provided, composed of two modular units 13 placed at 90° one to the other and at 45° with respect to the feeding line of vessels 3 to be inspected.

Both units 13 are each equipped with a standard emitter 5 and with a sensor 7.

It is clear that in this way shadow areas created by the concavity 9 of the vessels to be inspected are removed, since the area not detected by a sensor will be detected by the other sensor at 90° with respect to the first one.

It is clear that in case of failure of one of said sensors 7, a user can go on working, if he deems it adequate, with only one of them, always getting a 95% inspection as was the case in prior art.

## Claims

1. Non-destructive X-ray inspection apparatus for glass vessels and/or metal cans for food industry, comprising a feeding line for transporting said vessels and/or cans (3) through said apparatus, a pair of emitter means (5) and a pair of sensor means (7), **characterised by**
- a static structure (11) comprising two modular units (13), mutually placed at 90° and placed at 45° in relation to said feeding line wherein a first one of said emitter means (5) and a first one of said sensor means (7) are incorporated in a first modular unit (13) and a second one of said emitter means (5) and a second one of said sensor means (7) are incorporated in a second modular unit (13), said two modular units (13) being arranged so as to provide for said emitter means (5) on one side of said feeding line for emission of an X-ray beam and said sensor means (7) on the other side of said feeding line and facing said emitter means, so that X-rays collected by said sensor means (7) in said first modular unit (13) and X-rays collected by said sensor means (7) in said second modular unit (13) intersect said feeding line at an angle of 45° in a common crossing area, whereby said vessels and/or cans passing on said feeding line are inspected along two perpendicular directions in said common crossing area.

2. Inspection apparatus according to claim 1, wherein said apparatus can be used as a normal unitary structure for a 95% inspection using a single one of said modular units (13) at 45° with respect to said feeding line.

3. Inspection apparatus according to claim 1, wherein said apparatus with two emitters (5) and two sensors (7) arranged in corresponding modular units (13) provides the capability to inspect 100% of a product contained in said vessels and/or cans.

## Patentansprüche

1. Zerstörungsfreies Röntgenstrahluntersuchungsgerät für Glasbehälter und/oder Metalldosen für die Nahrungsmittelindustrie, das eine Förderstrecke zum Transport dieser Behälter und/oder Dosen (3) durch dieses Gerät und ein Paar von Sendeelementen (5) und ein Paar von Sensorelementen (7) aufweist, **gekennzeichnet durch**
- ein feststehendes Gerüst (11), das zwei modulare Einheiten (13) aufweist, die gegeneinander um 90° versetzt und in Bezug auf die Förderstrecke unter 45° platziert sind, wobei ein erstes Sendeelement (5) und ein erstes Sensorelement (7) in eine erste modulare Einheit (13) sowie ein zweites Sendeelement (5) und ein zweites Sensorelement (7) in eine zweite modulare Einheit (13) integriert sind und die beiden modularen Einheiten (13) so angeordnet sind, dass die Sendeelemente (5) auf einer Seite der Förderstrecke für die Emission eines Röntgenstrahlbündels und auf der anderen Seite der Förderstrecke die den Sendeelementen gegenüberliegenden Sensorelemente (7) vorgesehen sind, so dass Röntgenstrahlen, die **durch** die Sensorelemente (7) in der ersten modularen Einheit (13) aufgefangen werden, und Röntgenstrahlen, die von den Sensorelementen (7) in der zweiten modularen Einheit (13) gesammelt werden, die Förderstrecke unter einem Winkel von 45° in einer gemeinsamen Schnittebene schneiden, wobei die auf der Förderstrecke passierenden Behälter und/oder Dosen längs zweier senkrechter Richtungen in der gemeinsamen Schnittebene untersucht werden.

2. Untersuchungsgerät nach Anspruch 1, wobei das Gerät als normale Baueinheit zur 95%-Prüfung verwendet werden kann, indem nur eine der modularen Einheiten (13) unter 45° in Bezug auf die Förderstrecke genutzt wird.

3. Untersuchungsgerät nach Anspruch 1, wobei das Gerät mit jeweils zwei in den entsprechenden modularen Einheiten (13) angeordneten Sendern (5) und Sensoren (7) eine 100%ige Prüfung eines in den Behältern und/oder Dosen enthaltenen Produkts zu ermöglichen.

## Revendications

1. Appareil d'inspection non destructive par rayons X destinés à des récipients en verre et/ou à des boîtes métalliques destinés à l'industrie alimentaire, comprenant une ligne d'alimentation servant à transporter lesdits récipients et/ou lesdites boîtes (3) à travers ledit appareil, une paire de moyens émetteurs (5) et une paire de moyens détecteurs (7), **caractérisé par**
- une structure statique (11) comprenant deux unités modulaires (13), placées à 90° l'une de l'autre et à 45° par rapport à ladite ligne d'alimentation, dans lequel un premier desdits moyens émetteurs (5) et un premier desdits moyens détecteurs (7) sont incorporés dans une première unité modulaire (13) et un second desdits moyens émetteurs (5) et un second desdits moyens détecteurs (7) sont incorporés dans une seconde unité modulaire (13), les deux dites unités modulaires (13) étant agencées de façon à prévoir lesdits moyens émetteurs (5) sur un côté de ladite ligne d'alimentation pour une émission d'un faisceau de rayons X et lesdits moyens détecteurs (7) sur l'autre côté de ladite ligne d'alimentation et faisant face aux dits moyens émetteurs, de sorte que les rayons X collectés par lesdits moyens détecteurs (7) dans ladite première unité modulaire (13) et les rayons X collectés par lesdits moyens détecteurs (7) dans ladite seconde unité modulaire (13) coupent ladite ligne d'alimentation suivant un angle de 45° dans une zone de croisement commune, de sorte que lesdits récipients et/ou lesdites boîtes passant sur ladite ligne d'alimentation sont inspectés suivant deux directions perpendiculaires dans ladite zone de croisement commune.

2. Appareil d'inspection selon la revendication 1, dans lequel ledit appareil peut être utilisé sous la forme d'une structure unitaire normale pour une inspection à 95% en utilisant une seule unité desdites unités modulaire (13) à 45° par rapport à ladite ligne d'alimentation.

3. Appareil d'inspection selon la revendication 1, dans lequel ledit appareil doté de deux émetteurs (5) et de deux détecteurs (7) disposés dans des unités modulaires correspondantes (13) fournit la capacité d'inspecter 100% d'un produit contenu dans lesdits récipients et/ou lesdites boîtes.
